# EUROPÄISCHE PATENTANMELDUNG

(11) **EP 0 539 753 A1**
(43) Veröffentlichungstag der Anmeldung: **05.05.1993**
(21) Anmeldenummer: 92116819.1
(22) Anmeldetag: 01.10.1992
(51) Int. Cl.: G01N 11/12, G01N 33/32

(54) **Fallkörper-Viskosimeter**

(30) Priorität: 31.10.1991 DE 9113543 U
(71) Anmelder: FLEXACT MASCHINEN GmbH, D-51379 Leverkusen (DE)
(72) Erfinder: Putzke, Hans-Georg, W-5600 Wuppertal 1 (DE)
(74) Vertreter: Stark, Walter, Dr.-Ing.

(57) **Zusammenfassung**

Die Erfindung betrifft einen Fallkörperviskosimeter, insbesondere zur Bestimmung der Viskosität von Bäder aus Farben oder Farbmischungen. Ein präzise arbeitendes, auch größere Badmengen erfassendes Viskosimeter ist gekennzeichnet durch ein Gehäuse (2), eine im Gehäuse (2) in vertikaler Richtung geführte Stange (6), die am unteren Ende eine im Bad getauchte Fallscheibe (5) trägt, eine an der Stange (6) angreifende Hubeinrichtung (8), wenigstens einen Sensor (10) zum Erfassen der Fallstrecke oder der Fallzeit, eine mit einem Magnetventil (13) absperrbare Lösungsmittelzufuhr (12) sowie durch eine Steuereinrichtung (14), die die Hubeinrichtung (8) betätigt und nach Maßgabe der ermittelten Fallzeit das Magnetventil (13) steuert.

## Beschreibung

Die Erfindung betrifft ein Fallkörper-Viskosimeter, insbesondere zur Bestimmung der Viskosität von Bädern aus Farben oder Farbmischungen. Viskosimeter werden benötigt, um die Viskosität größerer Mengen von Farben oder Farbmischungen zu erfassen und durch Zusatz von Lösungsmitteln oder Farben konstant zu halten. Herkömmlich arbeitet man in diesem Zusammenhang mit Rührmeßsystemen, deren Genauigkeit jedoch zu wünschen übrig läßt.

Aufgabe der Erfindung ist es, ein präzise arbeitendes, auch größere Badmengen erfassendes Viskosimeter anzugeben.

Diese Aufgabe wird mit einem Fallkörper-Viskosimeter der eingangs beschriebenen Gattung gelöst, welches gekennzeichnet ist durch ein Gehäuse, eine im Gehäuse in vertikaler Richtung geführte Stange, die am unteren Ende eine im Bad getauchte Fallscheibe trägt, eine an der Stange angreifende Hubeinrichtung, wenigstens einen Sensor zum Erfassen der Fallstrecke oder der Fallzeit, eine mit einem Magnetventil absperrbare Lösungsmittelzuführung sowie durch eine Steuereinrichtung, die die Hubeinrichtung betätigt und nach Maßgabe der ermittelten Fallzeit das Magnetventil steuert. Dieses Viskosimeter arbeitet intermittierend, jedoch mit kurzen Zeitabständen. Z. B. kann alle 4 Sekunden eine Messung durchgeführt werden. Zu Beginn des Messvorganges wird die Stange mit der im Bad getauchten Fallscheibe um eine bestimmte Strecke angehoben. Sie bewegt sich dann unter Wirkung der Schwerkraft abwärts über eine Strecke von z. B. 1 cm. Das Ende der Fallstrecke wird vom Sensor erfaßt und nach Ermittlung der Fallzeit wird ggf. das Magnetventil aufgesteuert, um dem Bad eine bestimmte Menge Lösungsmittel zuzuführen.

In konstruktiver Hinsicht ist eine Ausführung vorteilhaft, bei der die Stange sich in einen Fallschacht erstreckt, der in einen das Bad enthaltenden Behälter eintauchbar ist und der im Bereich des unteren Endes wenigstens eine Öffnung aufweist, wobei die Fallscheibe sich im Bereich der Öffnung bewegt. Damit ist die Fallscheibe stets von der Badflüssigkeit umspült, die in üblicherweise umgewälzt wird. Insbesondere kann die Stange an Parallelogrammlenkern geführt sein, wobei die Hubeinrichtung an einem der Lenker angreift. Die Hubeinrichtung kann ein pneumatischer Hubzylinder sein. Der Sensor wird zweckmäßigerweise im Bereich der Stangenführung angeordnet. Die Lösungsmittelzufuhr kann grundsätzlich an beliebiger Stelle des Badbehälters erfolgen. Bei einer bevorzugten Ausführung der Erfindung ist das Magnetventil in einer Lösungsmittelleitung angeordnet, die in den Fallschacht mündet.

Bei einer bevorzugten Ausführung der Erfindung weist die Steuereinrichtung einen Soll/Ist-Vergleicher auf, der eine einstellbare Fall-Sollzeit mit der ermittelten Fall-Istzeit vergleicht und das Magnetventil oder einen Badzulauf steuert. Der Einstellbereich der Fall-Sollzeit kann den jeweiligen Verhältnissen angepasst werden. Für Farben oder Farbmischungen ist ein Einstellbereich von 14-35 Sekunden bei einer Genauigkeit von 1/10 Sekunden und einer Toleranz von +/- 0,4 Sekunden vorgegeben.

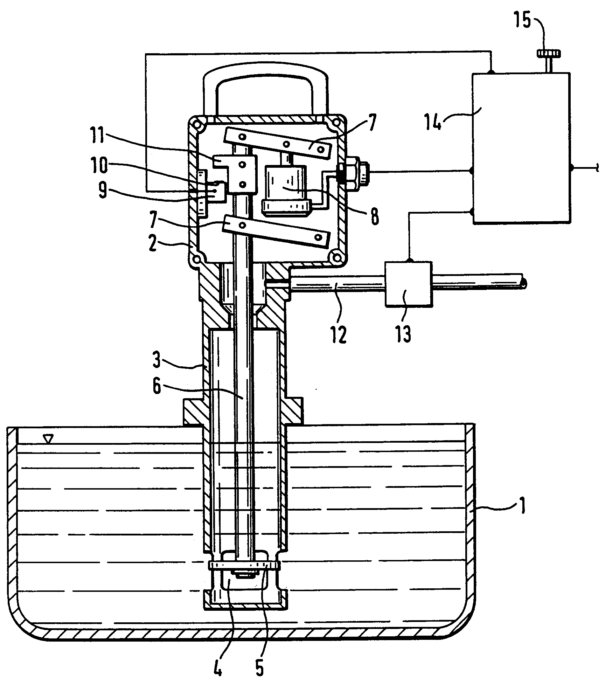

Im folgenden wird ein in der Zeichnung dargestelltes Ausführungsbeispiel der Erfindung erläutert; die einzige Figur zeigt in schematischer Darstellung ein Fallkörper-Viskosimeter.

Das in der Zeichnung dargestellte Fallkörper-Viskosimeter ist zur Bestimmung der Viskosität von Bädern aus Farben oder Farbmischungen bestimmt.

Das Bad aus Farben oder Farbmischungen befindet sich in einem Behälter 1 und wird darin umgewälzt. Der Behälter 1 weist einen nicht dargestellten Zulauf für Rohfarben und eine ebenfalls nicht dargestellte Entnahme für die Farbmischung auf. Das Fallkörper-Viskosimeter weist ein Gehäuse 2 und einen daran angeschlossenen Fallschacht 3 auf. Es wird so auf den Behälter 1 aufgesetzt, daß der Fallschacht 3 vertikal ausgerichtet in das Bad des Behälters 1 eintaucht. Der Fallschacht 3 weist am unteren Ende an seinem Umfang mehrere Öffnungen 4 auf, so daß zumindest dieses untere Ende des Fallschachtes 3 von der Badflüssigkeit durchspült wird. Im Bereich dieser Öffnungen 4 befindet sich eine Fallscheibe 5, die von einer Stange 6 getragen wird, welche sich durch den Fallschacht 3 bis in das Gehäuse 2 erstreckt und dort mit Hilfe von Parallelogrammlenkern 7 vertikal geführt ist. An einem der Parallelogrammlenker 7 greift ein pneumatischer Hubzylinder 8 an, mit dessen Hilfe das System aus Fallscheibe 5, Stange 6 und Parallelogrammlenkern 7 in eine vorbestimmte obere Position angehoben werden kann. Die untere Position dieses Systems wird bestimmt durch ein gehäusefestes Widerlageer 9, welches einen Sensor 10 trägt, und einen an der Stange 6 befestigten Anschlag 11.

In den Fallschacht 3 mündet eine Lösungsmittelleitung 12 mit einem darin angeordneten Magnetventil 13.

Zum Viskosimeter gehört außerdem eine Steuereinrichtung 14, die in regelmäßigen Abständen, z. B. alle 4 Sekunden, den Hubzylinder 8 aktiviert, damit das System aus Fallscheibe 5, Stange 6 und Parallelogrammlenkern 7 in die obere Position gebracht wird. Wenn die Kraft des Hubzylinders 10 nachläßt, sinkt das System ab, wobei die Fallzeit von der Viskosität der Badflüssigkeit nach der darin bewegten Fallscheibe 5 bestimmt wird. Da die Höhe der Öffnungen 4 am unteren Ende des Fallschachtes 3 etwas größer ist als die vorgegebene Fallstrecke der Fallscheibe 5, bewegt die Fallscheibe 5 stets in der umgewälzten Badflüssigkeit. Das Ende der Fallstrecke ist erreicht, wenn der Anschlag 11 auf das Widerlager 9 trifft und der Sensor 10 ein Signal an die Steuereinrichtung 14 abgibt. Die daraus ermittelte Fallzeit wird mit einem Soll/Ist-Vergleicher der Steuereinrichtung 14 zugeführt. An der Steuereinrichtung 14 kann bei 15 die Fall-Sollzeit eingestellt werden, bei der dargestellten Ausführung z. B. in einen Einstellbereich von 14 bis 35 Sekunden bei 1/10 Sekunden Genauigkeit und einer Toleranz +/- 0,4 Sekunden. Die gemessene Fall-Istzeit wird mit der Fall-Sollzeit verglichen. Bei zu langer Fall-Istzeit wird das Magnetventil 13 von der Steuereinrichtung 14 aufgesteuert, um der Badflüssigkeit Lösungsmittel zuzuführen. Bei zu kurzer Fall-Istzeit kann die Steuereinrichtung veranlassen, daß der Badflüssigkeit Rohfarbe zugeführt wird.

Nicht dargestellt ist, daß die Steuereinrichtung Anzeigeelemente für die Fall-Sollzeit, für die Fall-Istzeit sowie Anzeigen für fehlendes Lösungsmittel bzw. fehlende Farbe aufweist.

## Patentansprüche

1. Fallkörper-Viskosimeter, insbesondere zur Bestimmung der Viskosität von Bädern aus Farben oder Farbmischungen, gekennzeichnet, durch ein Gehäuse (2), eine im Gehäuse (2) in vertikaler Richtung geführte Stange (6), die am unteren Ende eine im Bad getauchte Fallscheibe (5) trägt, eine an der Stange (6) angreifende Hubeinrichtung (8), wenigstens einen Sensor (10) zum Erfassen der Fallstrecke oder der Fallzeit, eine mit einem Magnetventil (13) absperrbare Lösungsmittelzuführung (12) sowie durch eine Steuereinrichtung (14), die die Hubeinrichtung (8) betätigt und nach Maßgabe der ermittelten Fallzeit das Magnetventil (13) steuert.

2. Viskosimeter nach Anspruch 1, dadurch gekennzeichnet, daß die Stange (6) sich in einem Fallschacht (3) erstreckt, der in einen das Bad enthaltenden Behälter (1) eintauchbar ist und der im Bereich des unteren Endes wenigstens eine Öffnung (4) aufweist, wobei die Fallscheibe (5) sich im Bereich der Öffnung (4) bewegt.

3. Viskosimeter nach Anspruch 1 oder 2, dadurch gekennzeichnet, daß die Stange (6) an Parallelogrammlenkern (7) geführt ist und daß die Hubeinrichtung (8) an einem der Lenker (7) angreift.

4. Viskosimeter nach einem der Ansprüche 1 bis 3, dadurch gekennzeichnet, daß die Hubeinrichtung (8) ein pneumatischer Hubzylinder ist.

5. Viskosimeter nach einem der Ansprüche 1 bis 4, dadurch gekennzeichnet, daß der Sensor (10) im Bereich der Stangenführung angeordnet ist.

6. Viskosimeter nach einem der Ansprüche 1 bis 5, dadurch gekennzeichnet, daß das Magnetventil (13) in einer Lösungsmittelleitung (12) angeordnet ist, die in den Fallschacht (3) mündet.

7. Viskosimeter nach einem der Ansprüche 1 bis 6, dadurch gekennzeichnet, daß die Steuereinrichtung (14) einen Soll/Ist-Vergleicher aufweist, der eine einstellbare FallSollzeit mit der ermittelten Fall-Istzeit vergleicht und das Magnetventil (14) oder einen Badzulauf steuert.
